# EUROPEAN PATENT APPLICATION

(11) **EP 4 480 401 A1**
(43) Date of publication of application: **25.12.2024**
(21) Application number: 23180521.9
(22) Date of filing: 21.06.2023
(51) Int. Cl.: A61B 5/024, A61B 5/08, A61B 5/11, A61B 5/113, A61B 5/00

(54) **DEVICE, SYSTEM AND METHOD FOR DETECTING A DETERIORATION OF A SUBJECT`S HEALTH STATUS**

(71) Applicant: Koninklijke Philips N.V., 5656 AG Eindhoven (NL)
(72) Inventor: KIRENKO, Ihor Olehovych, 5656 AG Eindhoven (NL); ROCQUE, Mukul Julius, 5656 AG Eindhoven (NL)
(74) Representative: Philips Intellectual Property & Standards

(57) **Abstract**

A device for detecting a deterioration of a subject's health status comprises a respiratory signal extraction unit (41) configured to extract a respiratory signal related to the subject's respiration from first image data of the subject acquired by an imaging unit monitoring the subject, the first image data showing at least a first area of the subject's body allowing the extraction of a respiratory signal; a respiratory abnormality detection unit (42) configured to detect an abnormality in the extracted respiratory signal; a control unit (43) configured to generate, if an abnormality in the extracted respiratory signal has been detected, a control signal for controlling the imaging unit to change one or more settings of the imaging unit and to acquire second image data of the subject showing at least a second area of the subject's body allowing the extraction of a pulse signal related to the subject's heartbeat; a pulse rate extraction unit (44) configured to extract a pulse signal related to the subject's heartbeat from the second image data of the subject; and a deterioration detection unit (45) configured to detect a deterioration of the subject's health status based on the extracted respiratory signal and the extracted pulse signal.

## Description

### FIELD OF THE INVENTION

The present invention relates to a device, system and method for detecting a deterioration of a subject's health status.

### BACKGROUND OF THE INVENTION

Around 30%-40% of deteriorations in hospitals are happening in low acuity settings, and due to the lack of caregivers and monitoring systems, such deteriorations are not detected in time. With the growing pressure on hospitals budgets and lack of highly trained staff in high acuity areas such as ICUs, patients (or, more generally, subjects) will move to low acuity settings from ICU earlier, and the situation with a late detection of deteriorations may become even worse. Therefore, there is a growing need to provide early detection of deterioration, without significant investment into new patient monitoring equipment, clinical staff, and preferably using already installed equipment, or a low-cost commercial off-the-shelf hardware (COTS HW).

In the last years, devices, systems and methods as well as algorithms for contactless camera-based monitoring of vital signs (such as pulse rate (PR), respiration rate (RR) and oxygen saturation (SpOz) using (remote) photoplethysmography (rPPG)) and patient activity have been developed. Such algorithms, combined with camera hardware can provide semi-continuous and unobtrusive monitoring of patient conditions to enable early detection of deterioration in an automated way, without imposing additional burden on caregivers. However, a combination of those algorithms with COTS camera hardware to provide an optimal tradeoff between monitoring features and cost is not trivial.

### SUMMARY OF THE INVENTION

It is an object of the present invention to enable semi-continuous monitoring of vital signs and automatic detection of deterioration of a subject's health status without causing any costs or only low costs for necessary hardware.

In a first aspect of the present invention a device for detecting a deterioration of a subject's health status is presented comprising:
- a respiratory signal extraction unit configured to extract a respiratory signal related to the subject's respiration from first image data of the subject acquired by an imaging unit monitoring the subject, the first image data showing at least a first area of the subject's body allowing the extraction of a respiratory signal;
- a respiratory abnormality detection unit configured to detect an abnormality in the extracted respiratory signal;
- a control unit configured to generate, if an abnormality in the extracted respiratory signal has been detected, a control signal for controlling the imaging unit to change one or more settings of the imaging unit and to acquire second image data of the subject showing at least a second area of the subject's body allowing the extraction of a pulse signal related to the subject's heartbeat;
- a pulse rate extraction unit configured to extract a pulse signal related to the subject's heartbeat from the second image data of the subject; and
- a deterioration detection unit configured to detect a deterioration of the subject's health status based on the extracted respiratory signal and the extracted pulse signal.

In a further aspect of the present invention a system for detecting a deterioration of a subject's health status is presented comprising:
- an imaging unit configured to monitor the subject and acquire image data of the subject; and
- a device as disclosed herein for detecting a deterioration of the subject's health status based on the acquired image data.

In yet further aspects of the present invention, there are provided a corresponding method, a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed method, system, computer program and medium have similar and/or identical preferred embodiments as the claimed device, in particular as defined in the dependent claims and as disclosed herein.

The present invention is based on the principle of continuous monitoring of patient respiratory motion in order to detect moments when camera settings (e.g. view, zoom, resolution, etc.) should be modified to enable pulse monitoring or monitoring of other vital signs in a camera-based contactless way. Respiration monitoring and pulse monitoring can both be carried out based on image data acquired by an imaging unit (e.g. a camera) from the subject. However, instead of continuous monitoring of respiration, pulse and other vital signs, it is proposed according to the present invention to perform a continuous monitoring (or monitoring at a high rate) of respiration with intermittent monitoring of pulse if the respiration monitoring indicates an abnormality, e.g. a very high or very low respiration rate, an abnormal respiratory pattern, etc. The approach is making use of the fact that abnormal changes of respiration is the first sign of deterioration of the health condition, earlier than changes in pulse, SpOz, etc.

For instance, as provided in an exemplary embodiment, when RR extraction from the continuous monitoring of respiration gives an abnormal result, PR monitoring may be activated, e.g. by measuring ballistocardiographic motion after zooming into the chest area of the patient only, or by zooming into a skin area of a patient if visible light conditions allow PPG-based PR extraction. Hereby, the measurement of the vital signs related to respiration and pulse can be fed into existing patient monitoring system or early warning score (EWS) systems can make use of existing cameras, e.g. installed surveillance camera systems, and thus do not require additional hardware both for image acquisition and processing of acquired image data for extraction of several different vital signs.

According to an embodiment, the control unit is configured to generate a control unit for controlling the imaging unit to perform one or more of:
- zooming in to the second area of the subject's body;
- acquiring the second image data with a higher resolution and/or at a higher acquisition rate than the first image data;
- applying a digital zoom;
- adapting the exposure for the region of interest;
- increase the dynamic range; and
- reduce the compression.
This ensures that pulse-related information can be derived from the second image data, preferably with high reliability and accuracy and at a high rate and within a short time.

The control unit may further be configured to generate a control unit for controlling the imaging unit to zoom in to the chest area of the subject's body and
the pulse rate extraction unit may be configured to measure ballistocardiography motion from the second image data acquired from the chest area of the subject's body and to determine the pulse signal from the measured ballistocardiography motion. The use of ballistocardiography (BCG) motion for determining a pulse signal, in particular the pulse rate, is generally known in the art. This may be used to derive the pulse signal from the second image data.

According to another embodiment the control unit may be configured to generate a control unit for controlling the imaging unit to zoom in to a skin area of the subject's body and the pulse rate extraction unit may be configured to extract the pulse signal from the second image data acquired from the skin area of the subject by use of photoplethysmography. This embodiment makes use of the principles of photoplethysmography (PPG), according to volume changes of subcutaneous blood vessels caused by blood flow can be detected through the measurement of light reflected from skin in response to incident light (e.g. ambient light or light from a dedicated light source). Thus, PPG signals can be derived from the second image data, which then allow the extraction of a pulse signal or other pulse-related information.

The respiratory signal extraction unit may be configured to measure movements of the subject's chest, abdomen and/or diaphragm from the first image data and to determine the respiratory signal from measured movements. Such (generally periodic) movements reflect the respiration rate and may allow to determine the respiration pattern or even information regarding inhalation and/or exhalation volume.

There may generally be various abnormalities regarding the respiration that may be reflected in the respiratory signal and that may be interpreted as respiratory abnormality, which should cause the acquisition of pulse-related information. According to an embodiment, the respiratory abnormality detection unit is configured to detect the abnormality in the extracted respiratory signal by detecting a respiration rate above an upper respiration rate threshold or below a lower respiration rate threshold or a change of the respiration rate above a respiration rate change threshold or an abnormal breathing pattern. The various thresholds may be predetermined and fixed but may also be chosen for the particular subject or the type of subject (e.g., a patient group to which the subject belongs based on one or more of gender, age, weight, health status, kind of disease, disease level, etc.). An abnormal breathing pattern may be detected by a comparison of the respiratory signal or a breathing pattern derived there from to a reference breathing pattern, which may be a standardized / normal breathing pattern or a breathing pattern of the subject measured once the subject was healthy and showed a normal breathing pattern.

According to another embodiment the deterioration detection unit is configured to detect the deterioration of the subject's health status by detecting an abnormality in the extracted pulse signal and by determining that there is a deterioration of the subject's health status if an abnormality in the extracted respiratory signal and an abnormality in the extracted pulse signal have been detected. Thus, preferably in both the respiratory signal and the pulse signal, an abnormality should be seen to determine that the subject's health status is somewhat deteriorated. In other embodiments, a weighting may be applied to any (potential or real) abnormalities in both signals, optionally depending on one or more of the severity, duration, kind of abnormality, repetition rate of the abnormality, etc., of the abnormalities, when it is determined if the subject's health status is deteriorated.

The deterioration detection unit may further be configured to determine that there is a deterioration of the subject's health status if an abnormality in the extracted respiratory signal and an abnormality in the extracted pulse signal have been detected within a detection time interval, in particular within a predetermined detection time interval. The time interval may be defined by a caregiver according to clinical practice and/or the health status of the subject. In an exemplary implementation the detection time interval may have a duration in the range of 5 seconds to 10 minutes. For instance, if the abnormality in the pulse signal appears only 30 minutes after the abnormality in the respiratory signal, it may be determined that there is no deterioration in the subject's health status. Generally, the length of the detection time interval may be subject to implementation by the user and may much depend on the subject and/or the subject's general health condition and/or other criteria.

The deterioration detection unit may further be configured to detect the abnormality in the extracted pulse signal by detecting a pulse rate above an upper pulse rate threshold or below a lower pulse rate threshold or a change of the pulse rate above a pulse rate change rate threshold or an abnormal pulse pattern. Similar as explained above for the thresholds regarding respiration, these thresholds may be predetermined and fixed as well, or may be chosen for the particular subject or the type of subject. An abnormal pulse pattern may be detected by a comparison of the pulse signal or a pulse pattern derived there from to a reference pulse pattern, which may be a standardized / normal pulse pattern or a pulse pattern of the subject measured once the subject was healthy and showed a normal pulse pattern.

According to another embodiment the deterioration detection unit is configured to generate a warning score, an alert or a recommendation for a recommended course of action in case a deterioration of the subject's health status has been detected. The user, e.g. a caregiver or physician, may thus be directly and quickly alerted of the deterioration and thus can take an appropriate action, e.g. perform further measurements, start a treatment, check the measurement equipment, etc.

According to still another embodiment the control unit is configured to generate a control unit for controlling the imaging unit if an abnormality in the extracted respiratory signal and an abnormality in the motion of the subject or no motion at all have been detected. Thus, further criteria may be used to control the imaging unit, which provides a further level of safety that a deterioration of the subject's health status is quickly and reliably detected.

Still further, in an embodiment the control unit is configured to generate a control unit for controlling the imaging unit if an abnormality in the extracted respiratory signal and an abnormality in another respiratory signal acquired by a wearable or a contact sensor worn by the subject have been detected. This embodiment further improves reliability of the detection as well.

### BRIEF DESCRIPTION OF THE DRAWINGS

These and other aspects of the invention will be apparent from and elucidated with reference to the embodiment(s) described hereinafter. In the following drawings
Fig. 1 shows a schematic diagram of an embodiment of a system according to the present invention.
Fig. 2 shows a schematic diagram of a first embodiment of a device according to the present invention.
Fig. 3 shows diagrams of various signals used according to an embodiment of the present invention.
Fig. 4 shows a schematic diagram of a subject and different imaging areas for acquiring image data according to an embodiment of the present invention.
Fig. 5 shows a schematic diagram of a method according to the present invention.

### DETAILED DESCRIPTION OF EMBODIMENTS

Retrospective analyses of large electronic medical record (EMR) databases show the early deterioration detection performance using only RR and PR is substantially on par with the early deterioration detection performance of a full national early warning score system (NEWS) system using multiple physiological parameters in addition to RR and PR, such as oxygen saturation, temperature, systolic blood pressure and level of consciousness.

Such a system can be enabled by camera monitoring technology, but the potential for monitoring the pulse rate of patients continuously or almost continuously is challenging for multiple reasons: a) the performance accuracy of rPPG drops in night conditions due to lower SNR of infrared cameras at a distance; b) the detection of skin pixels on a sleeping patient, who can have very limited skin visibility due to camera orientation or due to covering of skin with blankets during sleeping, may be difficult or even impossible; 3) the extraction of a pulse signal from an image data (e.g. video data) using rPPG technology requires an access to uncompressed and not processed image data / videos, which is practically not possible or hardly possible in installed camera surveillance systems, where image data / videos are often streamed to a central entity (e.g. a central server) in a compressed, lower quality format to comply with network bandwidth limitations.

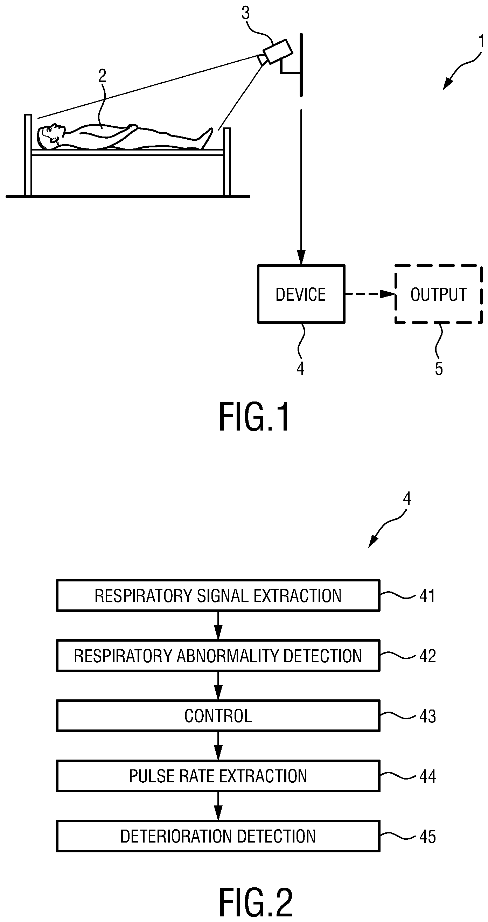

Fig. 1 shows a schematic diagram of an embodiment of a system 1 for monitoring a subject 2, e.g. patient in a hospital bed or an elderly person at home or in a rest home. The system 1 comprises an imaging unit 3 for monitoring the subject and acquiring image data of the subject. The system 1 further comprises a device 4 for detecting a deterioration of the subject's health status based on the acquired image data.

The imaging unit 3 may be a camera to record images (e.g. subsequent still images or video data) continuously or almost continuously, e.g. at a high frame rate. For instance, a surveillance camera or pan-tilt-zoom (PTZ) camera capable of high quality (optical) zoom into the patient room may be used, by which the SNR limitation may be overcome.

Details of the device 4 will be described below. The device 4 may be implemented by respective units or circuitry, e.g. a processor, processing circuitry, a computer, dedicated hardware, etc., that carries out the functions of the device. Alternatively, a common unit or circuitry, e.g. a common processor or computer, may implement the various functions of the device, or separate units or elements may be used that together represent the circuitry. In an exemplary implementation, a programmed processor may represent the device 4, which may execute a computer program that may be stored in a memory that is accessed by the processor.

The system 1 optionally further comprise an output interface 5 for issuing information regarding the detected deterioration of the subject's health status, e.g. a warning, a score, a recommendation (regarding a possible action), etc. Further, the output interface 5 may issue the extracted respiratory signal and the extracted pulse signal, e.g. an information about RR and PR. The output interface 5 may generally be any means that outputs the desired information in visual (or audible) form, e.g. in text form, as image or diagram, as sound or spoken words, etc. For instance, the output interface 5 may be a display, a loudspeaker, a touchscreen, a computer monitor, the screen of a smartphone or tablet, etc.

In a practical implementation, the device 4 and the output interface 5 may be implemented as or integrated into a patient monitor or other monitoring equipment.

Fig. 2 shows a schematic diagram of an embodiment of a device 4 for detecting a deterioration of the subject's health status according to the present invention.

The device 4 comprises a respiratory signal extraction unit 41 for extracting a respiratory signal related to the subject's respiration from first image data of the subject acquired by an imaging unit monitoring the subject. The first image data show at least a first area of the subject's body allowing the extraction of a respiratory signal. The first image data, e.g. video data, may directly be obtained (i.e. retrieved or received) from the camera 3, or from a storage, buffer, network, or bus, etc. The device 4 may thus be coupled with the camera 3 (or storage, buffer, etc.) by a (wired or wireless) communication or data link, such as a Bluetooth connection, Wi-Fi connection, LAN connection, HDMI connection, direct cable connection, or any other suitable connection allowing the image data transfer to the device 4.

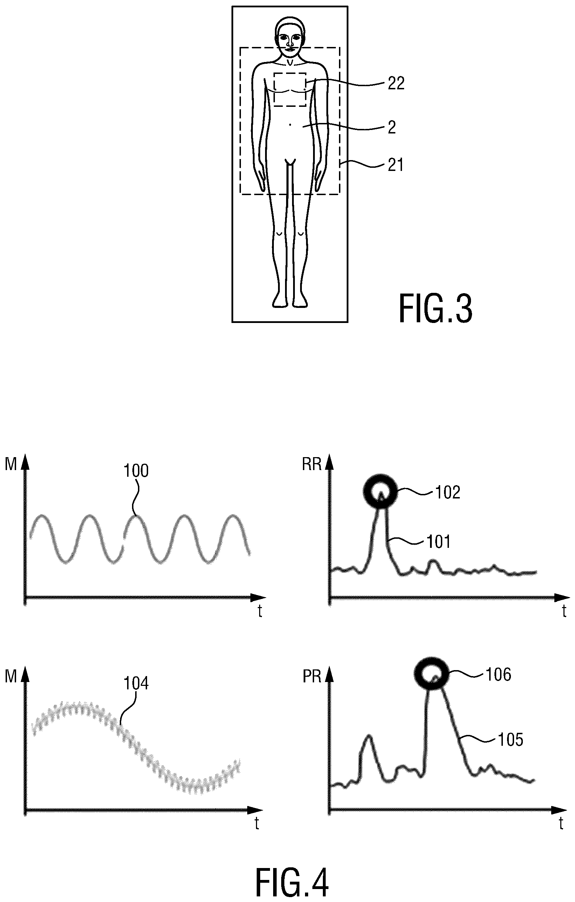

The first image data are preferably image data of a torso area (e.g. chest or abdomen) of the subject 2 from which the respiratory signal is extracted by monitoring and detecting movements of the chest wall or the diaphragm. Fig. 3 schematically shows a subject 2 and indicates a first imaging area 21, from which the first image data are acquired, i.e. the settings of the field of view (or zoom) of the imaging unit 3 are made accordingly. The respiratory signal may be the respiration rate or other respiratory information, such as breathing pattern, inhalation depth, exhalation depth, spatial uniformity of respiratory motion (e.g. chest motion), etc. The first image data may be compressed video data, which may generally enable the extraction of the respiratory information with sufficient accuracy and reliability.

Fig. 4 shows a chest movement signal 100 extracted from the first image data, i.e., it shows the up and down movement M of the chest wall over time t. The respiration rate signal 101 shows the respiration rate RR over time derived from the chest movement signal 100.

The device 4 further comprises a respiratory abnormality detection unit 42 for detecting an abnormality in the extracted respiratory signal. For instance, in the example of the respiration rate signal 101 shown in Fig. 4, an abnormality in the detected by detecting if the RR is too low (e.g. below a lower RR threshold) or too high (above an upper RR threshold). Such RR thresholds may be predetermined or set by a user and may be personalized to the particular subject or the group of subjects (e.g. according to age, gender, BMI, health status, etc.) to which the subject belongs. In Fig. 4 a too high RR is indicated by a circle 102. Other abnormalities may be an abnormal breathing pattern, non-periodic respiration, absence of respiration, long pauses between subsequent inhalations, low inhalation volume, etc.

The device 4 further comprises a control unit 43 for generating, if an abnormality in the extracted respiratory signal has been detected, a control signal for controlling the imaging unit 3 to change one or more settings of the imaging unit 3 and to acquire second image data of the subject showing at least a second area of the subject's body allowing the extraction of a pulse signal related to the subject's heartbeat. For instance, referring to Fig. 4, when the too high RR 102 is detected, the camera 3 is controlled to zoom in on the chest heart area 22 (see Fig. 3) of the subject 2 and to acquire second image data from the chest heart area 22, from which a ballistocardiography (BCG) motion signal may then be extracted.

Alternatively or additionally, the imaging unit 3 may be controlled to change the location of the field of view, i.e. to pan or tilt the imaging unit 3, and/or to change the resolution of the acquired second image data, e.g. to record second image data with a higher resolution than the first image data. In another embodiment, instead of the chest heart area 22, the imaging unit 3 may be controlled to zoom in on a skin area of the subject 2, e.g. a portion (e.g. cheek or forehead) of the face or throat (i.e. by focusing on an area above the chest from which the first image data have been acquired) or of the hand, and to acquire second image data from the skin area, from which a PPG signal can then be derived. In this embodiment, the skin area is sufficiently large to allow an extraction of a PPG-based pulse signal even from lossy compressed videos. In further embodiment, a digital zoom may be applied, the exposure for the region of interest may be adapted, the dynamic range may be increased, and/or the compression may be reduced to preserve more details.

The device 4 further comprises a pulse rate extraction unit 44 for extracting a pulse signal related to the subject's heartbeat from the second image data of the subject. The pulse signal may be obtained by deriving ballistocardiography motion caused by heart pulse from the second image data acquired e.g. from the chest heart area 102. Such a ballistocardiography motion signal represents a graphical representation of repetitive motions of the human body arising from the sudden ejection of blood into the great vessels with each heartbeat. Such motion B over time t is depicted in Fig. 4 as ballistocardiography motion signal 103. The pulse rate signal 104 shows the pulse rate PR over time derived from the ballistocardiography motion signal 103.

In an alternative embodiment the pulse signal may be obtained by deriving a PPG signal from the second image data acquired e.g. from a skin area. A PPG signal represents a graphical representation of changes in the volume of blood vessels in the subcutaneous tissue caused by the pressure pulse of blood pumped with each cardiac cycle from the heart to the periphery. The PPG signal represents the amount of light reflected from the skin portion in response to incident light (e.g. ambient light or light from an illumination unit), i.e., appropriate light conditions need to be present to make use of the option.

The device 4 further comprises a deterioration detection unit 45 configured to detect a deterioration of the subject's health status based on the extracted respiratory signal and the extracted pulse signal. For instance, based on a RR value and a PR value (acquired substantially at the same time), a preliminary warning score may be calculated based e.g. only on RR and PR, and optionally on further available physiological information. On detection of a severe deterioration based on the preliminary warning score, multiple vital sign spot check measurements and calculation of a full EWS may be initiated. In another embodiment, a deterioration may directly be detected from the RR and PR values, e.g. if both RR and PR show abnormal values or from both the respiratory signal and the extracted pulse signal, e.g. if both show abnormalities.

The units 41-45 of the device 4 may be implemented as a single or multiple processing unit(s). The processing unit(s) may be any kind of means configured to process the image data and determine the desired information there from. It may be implemented in software and/or hardware, e.g. as a programmed processor or computer or in the imaging unit 3 or a patient monitor or even as app on a user device such as a smartphone, smartwatch, tablet, laptop, PC, workstation.

The device 4 may optionally further comprise an output (not shown) configured to output information if a deterioration of the subject's health status has been detected, e.g. an indication that a deterioration has been detected, or a recommendation of actions to be performed, a warning or alarm, etc. The output may generally be any interface that provides the information, e.g. transmits it to another device or provides it for retrieval by another device (e.g. a smartphone, computer, tablet, central server or monitor, etc.). It may thus generally be any (wired or wireless) communication or data interface.

In another embodiment, monitoring of the pulse signal can be triggered not only by measuring a respiratory signal, but also by video actigraphy, e.g. by detecting an absence of a motion or a specific suspicious motion of a patient. Further, in an embodiment, monitoring of the pulse signal by a camera-based surveillance system can be triggered by a specific measurement provided by a wearable sensor worn by the patient. For instance, if a patient wears an accelerometer-based sensor (e.g. HealthDot which is a wearable biosensor that continuously captures the patient's heart rate, respiratory rate, posture, and activity and transmits these vitals every 5 minutes, on average, to the clinical information system), or a wearable PPG based smartwatch sensor, pulse signal measurement can be triggered by a specific respiratory pattern or abnormality from HealthDot.

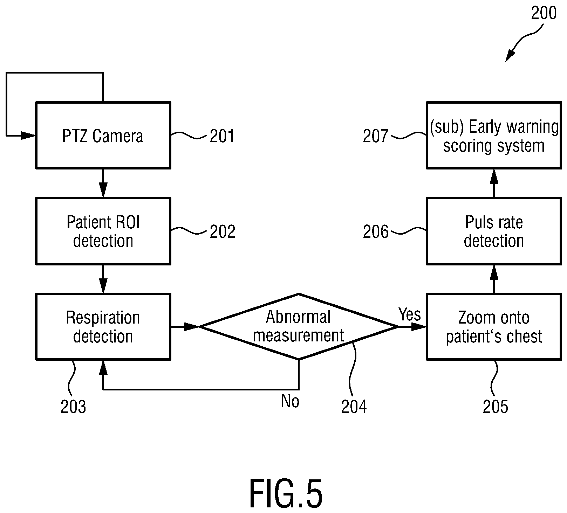

Fig. 5 shows a schematic diagram of an embodiment of a method 200 for detecting a deterioration of a subject's health status according to the present invention. The steps of the method may be carried out by the device 4. The method may e.g. be implemented as computer program running on a computer or processor.

In a first step 201, image data of a scene are acquired, e.g. by a PTZ camera, as long as no patient is detected in the scene. In a second step 202, a patient region of interest (ROI) is detected in the acquired image data, i.e. a patient is detected in the scene and a first imaging area (e.g. the chest area) is detected, from which first image data are acquired, e.g. after zooming in on the first imaging area. In a third step 203, a respiratory signal related to the subject's respiration is extracted from first image data of the subject. In a fourth step 204, an abnormality is detected in the extracted respiratory signal. If no abnormality is detected, the method proceeds with step 203. In a fifth step 205, if an abnormality in the extracted respiratory signal has been detected, a control signal is generated for controlling the imaging unit to change one or more settings of the imaging unit, e.g. to zoom in on a second imaging area, e.g. the patient's chest or a skin area of the patient, and to acquire second image data of the subject from the second imaging area. In a sixth step 206, a pulse signal related to the subject's heartbeat is extracted from the second image data of the subject. In a seventh step 207, a deterioration of the subject's health status is detected based on the extracted respiratory signal and the extracted pulse signal and a warning score, warning signal, recommendation or other relevant information for a user is generated.

In summary, health deterioration of patients in hospitals is often preceded by deterioration of physiological parameters. Resource limitations impose a limitation on the number of patients who can be monitored adequately in high acuity areas such as ICUs. With more patients having longer length of stays in low acuity settings such as general wards, where monitoring is very limited, health deteriorations of patients is an increasing problem in healthcare. An extended network of high quality PTZ cameras may be installed in multiple low acuity areas, however, requires additional monitoring infrastructure (such as patients monitors) and nurses to take multiple measurements to feed into EWS for clinical decision support. Such systems add additional burden to the overworked bedside clinicians, e.g. to initially prepare patients and subsequently perform multiple spot check measurements.

According to embodiments of the present invention, by the use of an imaging unit, such as a PTZ camera capable of high quality optical and/or digital zoom, SNR limitations may be overcome. Further, by using ballistocardiographic movements of the body due to the beating heart, by focusing on the chest of the patient, spot checks of pulse rate can be acquired. Since RR is known to be one of the most important early indicators of deterioration, RR may be continuously monitored at a high frequency (e.g. in the order of minutes), and only during abnormal RR measurements the camera zooms in on the chest to acquire a pulse rate (when the patient is not moving). Zooming in on the chest, close to the position of the heart, will increase the SNR of BCG based PR extraction. This achieves a balance between coverage of measurements and accuracy increase due to multiple physiological signal measurements.

The proposed invention can be used in hospital low acuity settings, nursing homes and elderly care facilities equipped with cameras, e.g. camera surveillance systems, to enable an early detection of deterioration. Further, in can be applied in virtual or remote monitoring systems that remotely monitor a patient.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. Device (4) for detecting a deterioration of a subject's health status, the device comprising:
- a respiratory signal extraction unit (41) configured to extract a respiratory signal related to the subject's respiration from first image data of the subject acquired by an imaging unit monitoring the subject, the first image data showing at least a first area of the subject's body allowing the extraction of a respiratory signal;
- a respiratory abnormality detection unit (42) configured to detect an abnormality in the extracted respiratory signal;
- a control unit (43) configured to generate, if an abnormality in the extracted respiratory signal has been detected, a control signal for controlling the imaging unit to change one or more settings of the imaging unit and to acquire second image data of the subject showing at least a second area of the subject's body allowing the extraction of a pulse signal related to the subject's heartbeat;
- a pulse rate extraction unit (44) configured to extract a pulse signal related to the subject's heartbeat from the second image data of the subject; and
- a deterioration detection unit (45) configured to detect a deterioration of the subject's health status based on the extracted respiratory signal and the extracted pulse signal.

2. Device as claimed in claim 1,
wherein the control unit (43) is configured to generate a control unit for controlling the imaging unit to perform one or more of:
- zooming in to the second area of the subject's body;
- acquiring the second image data with a higher resolution and/or at a higher acquisition rate than the first image data;
- applying a digital zoom;
- adapting the exposure for the region of interest;
- increase the dynamic range; and
- reduce the compression.

3. Device as claimed in claim 2,
wherein the control unit (43) is configured to generate a control unit for controlling the imaging unit to zoom in to the chest area of the subject's body and
wherein the pulse rate extraction unit (44) is configured to measure ballistocardiography motion from the second image data acquired from the chest area of the subject's body and to determine the pulse signal from the measured ballistocardiography motion.

4. Device as claimed in claim 2 or 3,
wherein the control unit is configured to generate a control unit for controlling the imaging unit to zoom in to a skin area of the subject's body and
wherein the pulse rate extraction unit (44) is configured to extract the pulse signal from the second image data acquired from the skin area of the subject by use of photoplethysmography.

5. Device as claimed in any preceding claim,
wherein the respiratory signal extraction unit (41) is configured to measure movements of the subject's chest, abdomen and/or diaphragm from the first image data and to determine the respiratory signal from measured movements.

6. Device as claimed in any preceding claim,
wherein the respiratory abnormality detection unit (42) is configured to detect the abnormality in the extracted respiratory signal by detecting a respiration rate above an upper respiration rate threshold or below a lower respiration rate threshold or a change of the respiration rate above a respiration rate change threshold or an abnormal breathing pattern.

7. Device as claimed in any preceding claim,
wherein the deterioration detection unit (45) is configured to detect the deterioration of the subject's health status by detecting an abnormality in the extracted pulse signal and by determining that there is a deterioration of the subject's health status if an abnormality in the extracted respiratory signal and an abnormality in the extracted pulse signal have been detected.

8. Device as claimed in claim 7,
wherein the deterioration detection unit (45) is configured to determine that there is a deterioration of the subject's health status if an abnormality in the extracted respiratory signal and an abnormality in the extracted pulse signal have been detected within a detection time interval, in particular within a predetermined detection time interval.

9. Device as claimed in claim 7 or 8,
wherein the deterioration detection unit (45) is configured to detect the abnormality in the extracted pulse signal by detecting a pulse rate above an upper pulse rate threshold or below a lower pulse rate threshold or a change of the pulse rate above a pulse rate change rate threshold or an abnormal pulse pattern.

10. Device as claimed in any preceding claim,
wherein the deterioration detection unit (45) is configured to generate a warning score, an alert or a recommendation for a recommended course of action in case a deterioration of the subject's health status has been detected.

11. Device as claimed in any preceding claim,
wherein the control unit is configured to generate a control unit for controlling the imaging unit if an abnormality in the extracted respiratory signal and an abnormality in the motion of the subject or no motion at all have been detected.

12. Device as claimed in any preceding claim,
wherein the control unit (43) is configured to generate a control unit for controlling the imaging unit if an abnormality in the extracted respiratory signal and an abnormality in another respiratory signal acquired by a wearable or a contact sensor worn by the subject have been detected.

13. System for monitoring a subject, the system comprising:
- an imaging unit (3) configured to monitor the subject and acquire image data of the subject; and
- a device (4) according to any preceding claims for detecting a deterioration of the subject's health status based on the acquired image data.

14. Method for detecting a deterioration of a subject's health status, the method comprising:
- extracting a respiratory signal related to the subject's respiration from first image data of the subject acquired by an imaging unit monitoring the subject, the first image data showing at least a first area of the subject's body allowing the extraction of a respiratory signal;
- detecting an abnormality in the extracted respiratory signal;
- generating, if an abnormality in the extracted respiratory signal has been detected, a control signal for controlling the imaging unit to change one or more settings of the imaging unit and to acquire second image data of the subject showing at least a second area of the subject's body allowing the extraction of a pulse signal related to the subject's heartbeat;
- extracting a pulse signal related to the subject's heartbeat from the second image data of the subject; and
- detecting a deterioration of the subject's health status based on the extracted respiratory signal and the extracted pulse signal.

15. Computer program comprising program code means for causing a computer to carry out the steps of the method as claimed in claim 14 when said computer program is carried out on the computer.
